# DEMANDE DE BREVET EUROPEEN

(11) **EP 1 000 625 A1**
(43) Date de publication de la demande: **17.05.2000**
(21) Numéro de dépôt: 99402701.9
(22) Date de dépôt: 29.10.1999
(51) Int. Cl.: A61K 35/74

(54) **Composition anti-bactérienne**

(30) Priorité: 30.10.1998 FR 9813703
(71) Demandeur: Laboratoire du Lacteol du Docteur Boucard, 78550 Houdan (FR); INSTITUT NATIONAL DE LA SANTE ET DE LA RECHERCHE MEDICALE (INSERM), 75013 Paris (FR)
(72) Inventeur: Servin, Alain, 92800 Nanterre (FR); Coconnier, Marie-Hélène, 94200 Ivry sur Seine (FR); Lievin, Vanessa, 92800 Nanterre (FR); Boisseau, Richard, 28260 Rouvres (FR); Houlier, Patrick, 75012 Paris (FR); Chauvierre, Gilles, 92160 Antony (FR)
(74) Mandataire: Ahner, Francis

(57) **Abrégé**

Le surnageant de culture de la souche *Lactobacillus acidophilus* LB est utilisé pour la préparation d'une composition dotée d'une activité anti-bactérienne contre *Helicobacter.* L'activité anti-bactérienne de ce surnageant est thermostable.

La composition contient en outre un support ingérable, notamment un support pharmaceutiquement acceptable et / ou un produit alimentaire. Elle est destinée à la prévention ou au traitement des affections causées par *Helicobacter.*

## Description

La présente invention concerne une nouvelle utilisation thérapeutique du surnageant de culture de la souche *Lactobacillus acidophilus* LB.

La souche *Lactobacillus acidophilus* LB, également dénommée souche Lactéol, est une souche microbienne d'origine humaine, connue pour ses propriétés antidiarrhéiques. Son mode d'action sur les principaux germes pathogènes impliqués dans les diarrhées a fait l'objet de nombreuses études (Servin et al., FEMS Microbiol. Lett. 1992, vol. 91, 213-218 ; Servin et al., J. Gen. Microbiol., 1992, vol. 138, 1689-1696 ; Servin et al. J. Diarrhoeal Dis. Res., 1993, 4, n°11, 235-242 ; Servin et al., FEMS Microbiol. Lett. 1993, vol. 110, 299-305).

On a mis en évidence chez l'animal, que la souche *Lactobacillus acidophilus* LB inhibe l'adhésion aux cellules intestinales des germes pathogènes *Escherichia coli* (Fourniat et al., Ann. Rech. Vét., 1986, 17, 401-407) et *Campylobacter jejuni* (Fauchère et al., Ann. Instit. Pasteur Microbiol., 1986, 137A, n°2, 199-207).

On sait désormais que le milieu de culture fermenté par *Lactobacillus acidophilus* LB a une action anti-bactérienne in vitro vis-à-vis des bactéries entérovirulentes *Staphylococcus* aureus, *Listeria monocytogenes, Salmonella typhimurium, Shigella flexneri, Klebsiella pneumonia, Escherichia coli, Bacillus cereus*, *Pseudomonas aeruginosa* et *Enterobacter* spp. En outre, le surnageant de culture de la souche *Lactobacillus acidophilus* LB exerce une activité anti-bactérienne sur la souche *Salmonella typhimurium* C5 chez des souris infectées par ce germe pathogène (A. Servin et al., Antimicrobial Agents and Chemotherapy, 1997, 1046-1052).

La souche GG de *Lactobacillus casei* a été décrite pour le traitement ou la prévention de la colite ulcérative et de la constipation. Son mécanisme d'action n'est pas connu mais EP-A2-199 535 suggère que la souche de *Lactobacillus casei* GG colonise l'intestin grâce à ses propriétés d'adhérence aux cellules intestinales, puis forme une barrière protectrice contre les pathogènes en produisant de l'acide lactique.

Il a été découvert dans le cadre de la présente invention que le surnageant de culture de la souche *Lactobacillus acidophilus* LB est dotée d'une activité anti-bactérienne contre *Helicobacter.*

On a déjà identifié dans l'art antérieur, une bactérie présentant une activité contre l'espèce *Helicobacter pylori :* il s'agit de *Lactobacillus johnsonii.* La souche CNCM 1-1225 de *Lactobacillus johnsonii* inhibe in vitro l'adhésion de *Helicobacter pylori* à un modèle de cellules intestinales (US 5 578 302).

Plus précisément, le surnageant de culture de *Lactobacillus johnsonii* (CNCM I-1225) provoque, in vitro, le déplacement compétitif des bactéries *Helicobacter pylori* adhérant à une culture monocouche de cellules intestinales HT-29. En effet, des 2.10⁶ cfu/cm² (colonies formant unités par centimètre carré) *Helicobacter pylori* adhérentes dénombrées en l'absence de *Lactobacillus johnsonii,* il n'en reste que 6.10³ cfu/cm² adhérentes après une incubation d'une heure avec *Lactobacillus johnsonii.*

La présente invention concerne l'utilisation d'un surnageant de culture de la souche *Lactobacillus acidophilus* LB pour la préparation d'une composition dotée d'une activité anti-bactérienne contre *Helicobacter.*

Dans le cadre de la présente invention, on entend par activité anti-bactérienne, en particulier toute activité capable de limiter la croissance bactérienne, notamment toute activité bactériostatique ou toute activité bactéricide.

De façon avantageuse, la composition de l'invention est dotée d'une activité anti-bactérienne contre *Helicobacter* après avoir exposé le surnageant de culture de la souche *Lactobacillus acidophilus* LB à une température comprise entre 90°C et 120°C pendant une période de 30 à 90 minutes, par exemple à 100°C pendant une heure.

La composition de l'invention peut être destinée à la prévention ou au traitement des affections causées par *Helicobacter.*

La composition de l'invention est en particulier capable d'exercer une activité anti-bactérienne contre *Helicobacter pylori,* principalement pathogène au niveau de l'estomac.

La composition de l'invention contient, outre le surnageant de culture de la souche *Lactobacillus acidophilus* LB, un support ingérable, en particulier un support pharmaceutiquement acceptable et / ou un produit alimentaire.

La figure 1 représente la variation de la viabilité de *Helicobacter pylori* (en ordonnée, log/ml) en fonction du temps (en abscisse, minutes) après ajout de MRS (courbe 1) ou du surnageant selon l'invention (courbe 2).

La figure 2 représente la mesure des *Helicobacter pylori* entéroadhérants viables (en ordonnée, log/ml) en fonction du temps (en abscisse, minutes), soumis au MRS (courbe 1) ou soumis au surnageant selon l'invention (courbe 2).

Les exemples suivants illustrent l'invention sans en limiter la portée.

### Exemple 1 : Viabilité de Helicobacter pylori in vitro

### Mise en culture de Helicobacter pylori :

*Helicobacter pylori* sont cultivés à 37°C pendant 18 heures dans des boîtes agar-BHI avec 0,25 % d'extrait de levure (DIFCO Laboratories), 10 % de sérum de cheval et 0,4 % d'un complément sélectif pour Campylobacter (Skirrow SR 69®, OXOID Ltd).

### Préparation du surnageant de la souche Lactobacillus casei GG

La souche *Lactobacillus casei* GG est cultivée dans un milieu De Man, Rogosa, Sharpe (MRS) de Biokar, pendant 18 heures à 37°C. Le surnageant est obtenu par centrifugation du milieu de culture ajusté à 5.10⁸ cfu/ml, pendant 30 minutes à 4°C et 10 000 g. Le surnageant centrifugé est filtré au moyen d'un filtre 0,22 µm. Le pH est ajusté à 4,5 avec une solution de HCl.

### Préparation du surnageant de la souche Lactobacillus acidophilus LB

Le contenu de 40 gélules de la spécialité Lactéol Fort® gélule est vidé dans 20 ml de tampon PBS. Après 10 min sous agitation magnétique, une centrifugation est pratiquée pendant 30 min à 5.000 tours/min. Le surnageant est récupéré et filtré sur papier filtre. Le pH de la solution est ajusté entre 4,7 et 5,3 avec une solution d'acide chlorhydrique à 0,1N ou d'hydroxyde de sodium 0,1N. Le surnageant de *Lactobacillus acidophilus* LB est obtenu par filtration stérilisante sur filtre 0,22 µm.

### Etude de viabilité de Helicobacter pylori in vitro

Une culture de *Helicobacter pylori* est remise en suspension dans du PBS et centrifugée à 4°C et 5 500 g pendant 5 minutes. Le surnageant est mis de côté ; les bactéries sont lavées avec du PBS stérile et remises en suspension dans du BHI.

La détermination du nombre de bactéries viables est effectuée en incubant approximativement 250 µl de *Helicobacter pylori* dans du BHI (4.10⁸ cfu/ml) avec 250 µl du surnageant de la souche *Lactobacillus acidophilus* LB, de *Lactobacillus johnsonii* LA 1 ou de *Lactobacillus casei* GG, et 500 µl de DMEM à 37°C. A intervalles de temps déterminés, des prélèvements sont effectués, successivement dilués et placés dans des boîtes agar-BHI pour compter les colonies de bactéries.

L'activité anti-bactérienne du surnageant de culture de la souche *Lactobacillus acidophilus* LB sur *Helicobacter pylori* est également mise en évidence sur des cellules intestinales humaines muco-sécrétantes dérivés d'adénocarcinomes HT29-MTX. Les HT29-MTX sont cultivés dans un milieu essentiel Eagle modifié par Dulbecco (DMEM), à 37°C sous une atmosphère à 10 % de CO₂ et 90 % d'air. On utilise les cellules après vingt jours de culture.

La mesure des *Helicobacter pylori* adhérants viables aux cellules HT29-MTX est effectuée lorsqu'on ajoute du MRS ajusté à pH 4,5 ou du surnageant de la souche *Lactobacillus acidophilus* LB.

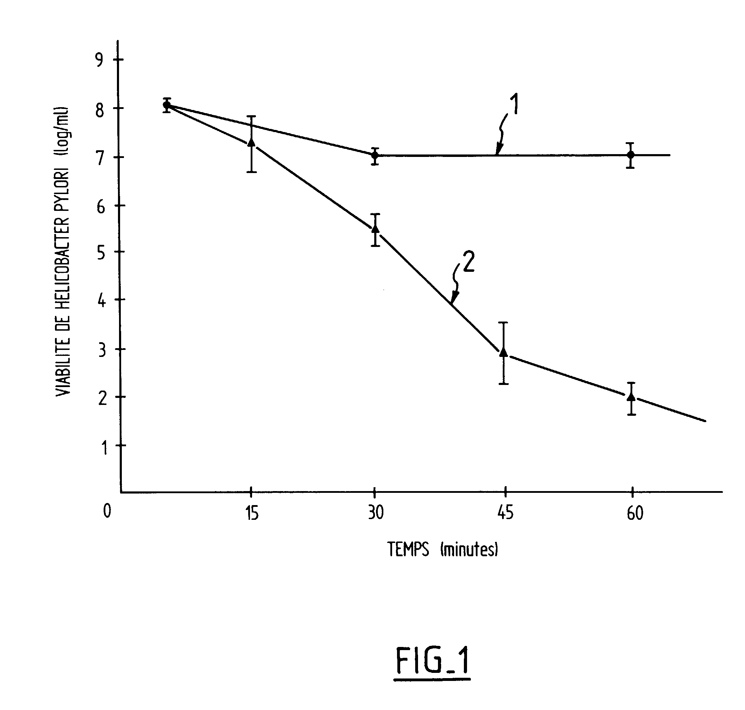

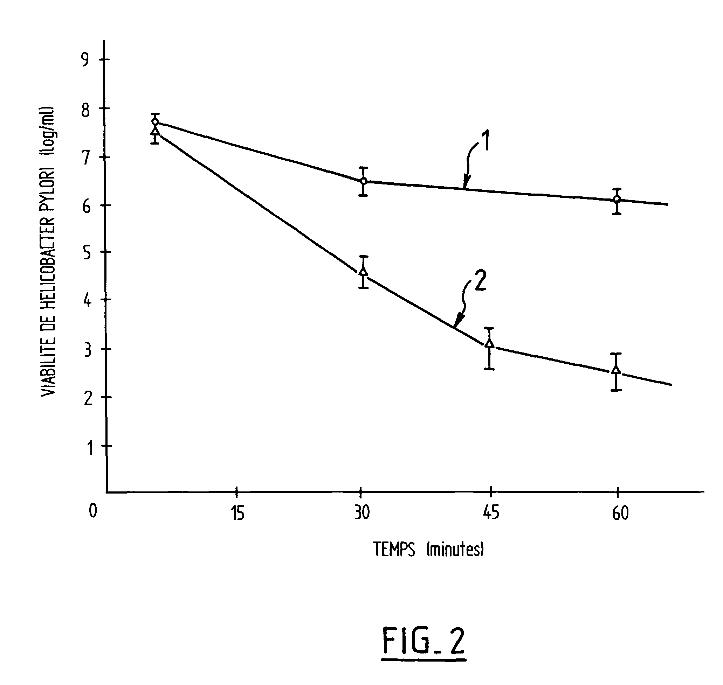

Les résultats sont présentés sur les figures 1 et 2.

Après 30 minutes et une heure, la viabilité de *Helicobacter pylori* soumis au surnanageant de *Lactobacillus acidophilus* LB chute respectivement de 2,5 et 6 unités log. La diminution de viabilité de *Helicobacter pylori* est dose-dépendante avec la quantité de surnageant de la souche *Lactobacillus acidophilus* LB ajoutée.

En ajoutant du surnageant de la souche *Lactobacillus casei* GG à la place du surnageant de la souche *Lactobacillus acidophilus* LB, on observe une diminution de la viabilité de *Helicobacter pylori* de 2,5 unités log au bout d'une heure, bien inférieure à la diminution observée avec *Lactobacillus acidophilus* LB.

Enfin, l'activité du surnageant de la souche *Lactobacillus acidophilus* LB après chauffage à 100°C pendant une heure est stable, tandis que celle du surnageant de la souche *Lactobacillus casei* GG et celle du surnageant de *Lactobacillus johnsonii LA 1* disparaissent pratiquement après chauffage.

Tous ces résultats sont présentés tableau 1.

**TABLEAU 1**

| Activité in vitro de MRS et des surnageants des souches Lactobacillus acidophilus LB, *Lactobacillus johnsonii* LA 1 et Lactobacillus *casei* GG contre *Helicobacter pylori.* | |
|---|---|
| | **Viabilité de *Helicobacter pylori* (log cfu/ml)** |
| **Référence** | 7,4 ± 0,2 |
| **MRS** | 6,9 ± 0,3 |
| **Surnageant de la souche *Lactobacillus acidophilus* LB** | 1,9±04 |
| **Surnageant de la souche *Lactobacillus acidophilus* LB après chauffage à 100°C pendant 1 heure** | 2,9 ± 0,4 |
| **Surnageant de la souche *Lactobacillus casei* GG** | 3,9 ± 0,7 |
| **Surnageant de la souche *Lactobacillus casei* GG après chauffage à 100°C pendant 1 heure** | 5,8 ± 0,4 |
| **Surnageant de *Lactobacillus johnsonii LA 1*** | 3,83 ± 0,45 |
| **Surnageant de *Lactobacillus johnsonii LA 1* après chauffage à 100°C pendant 1 heure** | 5,46 ± 0,40 |
| **Acide lactique DL** | 7.52 ± 0.08 |

Le pH est ajusté à 4,5 pour chaque mesure.

### Exemple 2 : Viabilité de Helicobacter felis in vivo chez la souris

### Mise en culture de Helicobacter felis :

*Helicobacter felis* sont cultivés à 37°C pendant 18 heures dans des boîtes agar-BHI avec 0,25 % d'extrait de levure (DIFCO Laboratories), 10 % de sérum de cheval et 0,4 % d'un complément sélectif pour *Campylobacter* (Skirrow SR 69®, OXOID Ltd).

Trois groupes de 12 à 13 souris sont infectés par voie intragastrique avec une concentration fixée de *Helicobacter felis* (0,2 ml, 10⁸ cfu/souris).

### Etude de viabilité de Helicobacter felis :

Trois semaines après l'infection, deux groupes de souris infectées sont traités durant sept jours. Chaque souris du premier groupe reçoit 1ml de surnageant de la souche *Lactobacillus acidophilus* LB. Chaque souris du second groupe reçoit 0,2 ml d'un mélange d'anti-acide et d'antibiotique en solution aqueuse (0,4 mg/ml d'Oméprazole et 62,5 mg/ml d'Amoxicilline).

Un jour et 42 jours après le traitement (respectivement 29^{ème} et 70^{ème} jours après l'infection), six à sept souris de chacun des trois groupes sont sacrifiées.

On effectue une analyse histologique sur l'estomac de chaque souris.

L'existence d'une inflammation et/ou d'une gastrite dans les différentes zones de la paroi gastrique est définie par la présence d'une infiltration de lymphocytes ou de neutrophiles ou par la présence d'une dédifférenciation des cellules épithéliales.
• Premier groupe : sans traitement (13 souris)
Au bout du 29^{ème} jour après l'infection, cinq des sept souris présentent les signes d'une inflammation gastrique. 70 jours après l'infection, quatre des six souris restantes présentent encore les signes de l'infection.
• Deuxième groupe : traitement avec le surnageant de la souche *Lactobacillus acidophilus* LB (12 souris).
Chez toutes les souris, un et 42 jours après le traitement, on détecte la présence de *Helicobacter felis* dans l'estomac.
Cependant, le nombre de *Helicobacter felis,* 1 jour après le traitement, mesuré sur les souris du deuxième groupe est beaucoup plus faible que chez les souris du premier groupe (souris non traitées), et aucune des souris traitées avec le surnageant de la souche *Lactobacillus acidophilus* LB ne présente des signes d'inflammation gastrique un jour après le traitement.
42 jours après le traitement, le nombre de *Helicobacter felis* est inchangé et tous les estomacs sont non inflammés.
• Troisième groupe : traitement avec Amoxicilline et Oméprazole (12 souris).
Le premier jour après traitement, toutes les souris sont complètement débarrassées de *Helicobacter felis* et aucune lésion inflammatoire n'est observée.
Au bout du 42^{ème} jour, *Helicobacter felis* réapparaît dans l'estomac des souris qui ne présentent cependant aucun signe histologique d'inflammation.
Les résultats sont résumés dans le tableau 2.

**TABLEAU 2**

| Analyse histopathologique et évaluation du nombre de *Helicobacter felis* chez des souris infectées avec *Helicobacter felis* puis traitées avec le surnageant de la souche *Lactobacillus acidophilus* LB ou un mélange Amoxicilline-Oméprazole. | | | |
|---|---|---|---|
| **Traitement** | **Jour du sacrifice** | **Nombre de souris présentant une gastrite et/ou une inflammation** | **Evaluation du nombre de *Helicobacter felis**** |
| **Référence** | 29 (7 souris) | 5/7 | 2.5 ± 0,4 |
| | 70 (6 souris) | 4/6 | 1.7 ± 0,4 |
| **Surnageant de la souche Lactobacillus acidophilus LB** | 29 (6 souris) | 0/6 | 1.5 ± 0,2 |
| | 70 (6 souris) | 0/6 | 1.5 ± 0,2 |
| **Amoxicilline + Oméprazole** | 29 (6 souris) | 0/6 | 0 |
| | 70 (6 souris) | 0/6 | 1.2 ± 0,3 |

| | | | |
|---|---|---|---|
| * Le nombre de *Helicobacter felis* a été indexé de 0 à 4. | | | |

0 : absence de bactérie
1 : bactéries isolées
2 : faible nombre de bactéries
3 : grand nombre de bactéries
4 : nombre élevé de bactéries.

Il ressort des deux exemples précédents que le surnageant de culture de la souche *Lactobacillus acidophilus* LB diminue considérablement la viabilité de *Helicobacter pylori* in vitro.

En outre, le traitement oral de souris avec un surnageant de la souche *Lactobacillus acidophilus* LB protège contre une infection par *Helicobacter felis.* Huit et quarante neuf jours après le début du traitement, on constate l'inhibition de la colonisation de l'estomac par *Helicobacter felis ;* aucune lésion histopathologique de l'estomac n'est observée.

Enfin, la souche *Lactobacillus acidophilus* LB sécrète des composés antibactériens qui s'avèrent in vitro plus efficaces que ceux de la souche *Lactobacillus casei* GG contre *Helicobacter pylori.*

## Revendications

1. Utilisation d'un surnageant de culture de la souche *Lactobacillus acidophilus* LB pour la préparation d'une composition dotée d'une activité anti-bactérienne contre *Helicobacter.*

2. Utilisation selon la revendication 1 caractérisée en ce que la composition est dotée d'une activité anti-bactérienne contre *Helicobacter* après avoir exposé le surnageant de culture à une température comprise entre 90°C et 120°C pendant une période de 30 à 90 minutes.

3. Utilisation selon les revendications 1 ou 2 caractérisées en ce que la composition est destinée à la prévention ou au traitement des affections causées par *Helicobacter.*

4. Utilisation selon l'une des revendications 1 à 3 caractérisée en ce que la composition possède une activité anti-bactérienne contre *Helicobacter pylori.*

5. Utilisation selon l'une des revendications de 1 à 4 caractérisée en ce que la composition comprend un produit alimentaire.

6. Utilisation selon l'une des revendications 1 à 5 caractérisée en ce que la composition comprend un support pharmaceutiquement acceptable.
